# EUROPÄISCHE PATENTANMELDUNG

(11) **EP 1 093 824 A2**
(43) Veröffentlichungstag der Anmeldung: **25.04.2001**
(21) Anmeldenummer: 00122690.1
(22) Anmeldetag: 18.10.2000
(51) Int. Cl.: A61L 15/42, A61L 15/58, A61L 31/06

(54) **Gefärbte Materialien aus bioabbaubaren Polymeren im medizinisch-pharmakologischen Bereich**

(30) Priorität: 21.10.1999 DE 19950646
(71) Anmelder: MERCK PATENT GmbH, 64293 Darmstadt (DE)
(72) Erfinder: Burkhard, Ingrid Dr., 64839 Münster (DE); Pfefferle, Hans, 64297 Darmstadt (DE)

(57) **Zusammenfassung**

Die Erfindung betrifft bioabbaubare Polymere geeignet als Wundbehandlungsmittel, die mit einem Farbstoff versehen sind und somit im Wundgebiet eindeutig erkannt werden können sowie deren Verwendung als Adhäsiv, Antiadhäsionsprophylaxe, Wundfolie oder Schaum.

## Beschreibung

Die Erfindung betrifft bioabbaubare Polymere geeignet als Wundbehandlungsmittel, die mit einem Farbstoff versehen sind.

Es besteht ein vielfältiger Bedarf an schützenden, abdichtenden bzw. klebenden Materialien im Bereich der medizinischen Wundheilung. Grundsätzlich haben solche Materialien zwei Hauptmerkmale zu erfüllen: Zum einen müssen die Eigenschaften des Materials den Anforderungen des bestimmungsgemäßen Einsatzes entsprechen, zum anderen sollte eine gute Verträglichkeit der Stoffe durch den menschlichen Organismus gewährleistet sein.
Aufgabe der Erfindung war es nun, Materialien zu entwickeln, die neben einer hohen Biokompatibilität und der Erfüllung ihrer bestimmungsgemäßen Eigenschaften sowie ihrer Biodegradierbarkeit eine optimale Anwendung im Wundgebiet ermöglichen.
Diese Aufgabe konnte dadurch gelöst werden, daß biokompatiblen polymeren Materialien wie z.B. einer Folie aus Copolylactid ein Farbstoff zugesetzt wird, wodurch die Anwendung z.B. als Antiadhäsionsprophylaxe im Wundgebiet entscheidend verbessert wird. Eine durchsichtige Folie hingegen wäre im Wundgebiet schlechter auffindbar.
Werden die Polymere als Adhäsiv für die Fixierung einer Wundabdeckungsfolie genutzt, kann der Anwender die Aufbringung eines durchsichtigen Klebers schlecht kontrollieren und damit wäre ein vollständiger Wundverschluß nicht gewährleistet. Dies ist aber zum Schutz gegen eine Superinfektion von entscheidender Bedeutung. Somit dient eine Anfärbung des Adhäsivs auch hier der Verbesserung der Anwendung.

Grundsätzlich können alle bekannten resorbierbaren polymeren Materialien verwendet werden. Bevorzugt sind Copolylactide bestehend aus D,L-Lactid und ε- Caprolacton.

Geeignete Lösungsmittel für das Lösen des Polymers sind Ethylacetat, Aceton, Dichlormethan und Chloroform.
Als Farbzusätze kommen alle biokompatiblen Farbstoffe wie z.B. D&C Green Nr. 6 in Frage.

Anhand des folgenden Beispiele wird die Erfindung näher erläutert:
Die Herstellung der im folgenden beschriebenen Copolymere ist aus dem Stand der Technik bekannt (siehe z.B. EP-Anmeldung Nr. 99116119.1 (Merck Patent GmbH)).

### Beispiel 1: Herstellung einer gefärbten Antiadhäsionsfolie

Der Farbstoff D&C Green Nr. 6 wird in einer Konzentration von 0.075 % in Ethylacetat gelöst. In diesem nun farbigem Lösungsmittel wird ein Copolylactid bestehend aus einem 50 : 50-Gemisch der Copolymere 85 Gew. % D,L- Lactid / 15 Gew. % ε-Caprolacton und 82 Gew. % D,L-Lactid / 18 % ε-Caprolacton in einer Konzentration von 20 Gew.% gelöst. Diese Copolylactidlösung wird nun z.B. mittels bekannter Rakeltechnik zu einer Folie weiterverarbeitet. Man erhält eine Folie aus biodegradierbarem, biokompatiblen und farbigen Material, die im Wundgebiet eindeutig erkannt werden kann.

### Beispiel 2: Herstellung eines gefärbten Adhäsivs

Der Farbstoff D&C Green Nr. 6 wird in einer Konzentration von 0.03 Gew. % in Ethylacetat gelöst. In diesem farbigen Lösungsmittel wird ein Copolylactid bestehend aus 85 Gew. % D,L-Lactid und 15 Gew. % ε-Caprolacton in einer Konzentration von 6 Gew. % gelöst. Man erhält eine niedrigviskose, klebrige Masse, die als Adhäsiv auf der Haut verwendet werden kann.

Durch die Färbung ist eine deutliche Markierung der bestrichenen Fläche möglich.

In gleicher Weise können für die Verwendung als Wundfolie oder als Schaum die entsprechend hergestellten Lösungen Einsatz finden.

## Patentansprüche

1. Bioresorbierbares Polymer geeignet als Wundbehandlungsmittel, dadurch gekennzeichnet, daß es mit einem Farbstoff versehen ist.

2. Bioresorbierbares Polymer gemäß Anspruch 1, dadurch gekennzeichnet, daß der Farbstoff D&C Green Nr. 6 ist.

3. Verwendung des Polymers nach einen der Ansprüche 1 und/ oder 2 als Antiadhäsionsprophylaxe.

4. Verwendung des Polymers nach einen der Ansprüchen 1 und/ oder 2 als Adhäsiv.

5. Verwendung des Polymers nach einen der Ansprüchen 1 und/ oder 2 als Wundfolie.

6. Verwendung des Polymers nach einen der Ansprüchen 1 und/ oder 2 als Schaum.
